# EUROPEAN PATENT APPLICATION

(11) **EP 2 939 620 A1**
(43) Date of publication of application: **04.11.2015**
(21) Application number: 13868640.7
(22) Date of filing: 17.11.2013
(51) Int. Cl.: A61B 17/34

(54) **WORKING CASING PIPE FOR MINIMALLY INVASIVE SURGERY**

(30) Priority: 30.12.2012 CN 201210585420
(71) Applicant: Shanghai Linchao Medical Instrument Co., Ltd., Shanghai 201318 (CN)
(72) Inventor: LIN, Chao, Shanghai 201318 (CN)
(74) Representative: Cabinet Laurent & Charras
(86) International application number: PCT/CN2013/087285
(87) International publication number: WO 2014/101581

(57) **Abstract**

A working casing pipe for minimally invasive surgery comprises a sheath (2), a silicone casing pipe (6), a multi-channel cap (9), and a multi-channel cushion (11). The multi-channel cap (9) and the multi-channel cushion (11) can be used for letting multiple instrument channels (8) pass through. Openings used for letting carbon dioxide channels (7) pass through are disposed on the multi-channel cap (9) and the multi-channel cushion (11). Multiple tracks (10) are disposed on the multi-channel cap to let the instrument channels (8) move left and right. Further, a gas injection hole (14) or a valve is disposed in the multi-channel cap (9) to fill gas into a gasbag (15) at the lower end of the silicone casing pipe (6). The multi-channel cushion (11) is corrugation-shaped, and the cross section may be V or U-shaped. The sheath (2) and the silicone casing pipe (6) are fitted together to fonn the middle part of the working casing pipe with both hard and soft materials. A penetrating channel (5) is located at the tail of the sheath (2) to let a penetrating knife pass through, and an angle is formed between the tail of the sheath (2) and the middle part of the sheath (2). The working casing pipe provides the possibility of direct penetration, enough support force and large operating space. It is flexible in operation and has functions of gas leakage preventing and coming-out preventing, thereby breaking the restriction of the existing working casing pipe on types and methods of the minimally invasive surgery and meeting requirements in complicated minimally invasive surgery.

## Description

### FIELD OF THE INVENTION

The present invention relates to a minimally invasive surgical instrument, particularly to a surgical working casing pipe used in the minimally invasive surgery, such as laparoscopy.

### BACKGROUND OF THE INVENTION

In the field of surgery, open surgery was usually used at home and abroad in the past. However, open surgical resection cause severe trauma and much bleeding, and the patient suffers great pain and slow recovery. While the minimally invasive surgery leaves small wound and little bleeding, and the patient undergoes little pain and relatively fast recovery. Therefore, with the development of medical science and technology, the open surgical resection is gradually replaced by the minimally invasive surgery. For example, with the laparoscopy, a patient's abdominal wall is penetrated or gets a small incision, then a laparoscopic instrument is placed inside through a penetration working casing pipe while the abdominal cavity or peritoneum cavity is filled with gas, and organ resection or repair surgery is completed by monitoring with a TV. The laparoscopy is an important milestone in the medical history of the 20th century.

In the minimally invasive surgery appearing in recent years, in order to reduce the patient's trauma on the abdominal wall and in consideration of the postsurgical esthetic effect, for example, the abdominal wall is penetrated around the navel with a working casing pipe, and a laparoscope and two instruments are placed inside, which can facilitate the surgery, such as intraperitoneal cholecystectomy and nephrectomy by the intraperitoneal route. However, a single port penetrating casing pipe can be placed inside only by incising layers of the abdominal wall tissues, and the entirely soft puncture outfit is weak in supporting the incision, such as an incision of 3.5 cm. The incision channel for receiving the entirely soft puncture outfit has an operating space less than 3 cm; a hard puncture outfit can enlarge the incision from 3 cm to 3.5 cm, so as to increase the operating space, but the hard tubular puncture outfit makes the operation extremely inconvenient. When a doctor operates with both hands at the same time, due to limitation of the operating space, the instruments that enter the abdominal cavity through the single port penetrating casing pipe are easy to interfere with and obstruct each other, which is very inconvenient. Besides, because a third surgery instrument cannot be put inside, methods and types of the surgery are very limited, disenabling more complicated surgery to be completed. In order to facilitate bimanual operation, the aperture of the circular working casing pipe also needs to be increased, which will accordingly increase injury to the abdomen.

### CONTENTS OF THE INVENTION

A technical problem to be solved by the present invention is to provide a working casing pipe for minimally invasive surgery, which can overcome the above defects of the existing working casing pipe, and meet requirements of various types and methods of the minimally invasive surgery.

Another technical problem to be solved by the present invention is to provide a safer working casing pipe having more operating space and flexibility.

In order to attain the above purposes, the following technical solution is adopted: a working casing pipe with a penetrating channel used for a single port laparoscope, comprising a sheath, a silicone casing pipe, a multi-channel cap, and a multi-channel cushion.

Wherein the sheath and the silicone casing pipe are fitted together; the multi-channel cap is connected with the silicone casing pipe; and the multi-channel cushion is located below the multi-channel cap.

Preferably, with the sheath including three parts, the upper end of the sheath has a circular cross section and used for connection with the multi-channel cap, the middle part of the sheath has a semicircular cross section, and the tail of the sheath at the lower end is provided inside with a penetrating channel to form an insertion port for a penetrating knife.

Preferably, the semicircular middle part of the sheath is hard, and the silicone casing pipe is soft.

Preferably, the silicone casing pipe is soft and the sheath is hard; the said sheath and the silicone casing pipe are fitted together to form the middle part with both hard wall and soft wall of the working casing pipe.

Preferably, an angle is formed between the semicircular middle part and the tail of the sheath.

Preferably, the multi-channel cap is provided with multiple instrument channels and tracks to allow the instrument channels to move laterally. Each operating channel can move along the track, with the shape and number of the operating channel and sliding track determined according to the requirements of the surgery. For example, multiple operating tracks can be arranged in parallel, wherein each track can be arranged in a shape similar to a rectangle or a semicircle, and each track can receive one or more operating channels. The shape of the track can be determined as required, such that the instrument channel can be distributed flexibly in the multi-channel cap, so as to meet the requirements of operation of multiple instruments.

Preferably, the multi-channel cap is further provided with a carbon dioxide channel.

Preferably, the silicone casing pipe is provided at the lower end with a gasbag, so as to prevent the working casing pipe from coming out of the incision.

Preferably, the multi-channel cap includes an anti-reflux gas injection hole or valve for filling gas into the gasbag at the lower end of the silicone casing pipe.

Preferably, the multi-channel cushion, located below the multi-channel cap, is a soft elastomer, and provided with a carbon dioxide channel and multiple instrument channels that are connected with and in correspondence to the channels in the multi-channel cap.

Preferably, the multi-channel cushion is corrugation-shaped, and can stretch or shrink with movement of the operating channel, thus isolating the middle part of the casing pipe from the air outside the cap.

Preferably, the corrugation of the multi-channel cushion is, for example, an accordion-type corrugation, and has a V or U-shaped cross section.

Preferably, the operating channel is provided at the lower end with a duckbill anti-gas leakage flap.

Therefore, the working casing pipe for minimally invasive surgery provided by the present invention has a smaller aperture, multiple operating channels, larger operating space, enough support force, and a penetrating channel. Because it has a penetrating channel to allow a penetrating knife to pass through, and has sufficient support force for the incision, it can be penetrated directly into the body cavity after carbon dioxide is injected, not necessary to incise each layer of the tissue, not inclined to damage the organs in the body cavity, having small wound and being relatively safe. With multiple operating channels, multiple operating instruments can be received, more surgical methods and types can be used, and the requirements of more complicated surgery can be met. The multi-channel cap is provided with sliding tracks for the operating channel, and each operating channel can move along its corresponding track; the shape and number of the operating channels and sliding tracks can be determined according to the requirements of the surgery, such that the operation can be coordinated among the surgical instruments, avoiding the situation of mutual interference and obstruction. The hard middle part of the sheath and the soft silicone casing pipe are fitted together to form the middle part of the working casing pipe, which can not only support the largest operating space of the instrument , but also provide operation flexibility. A multi-channel cushion, provided below the multi-channel cap, is soft corrugation-shaped, and can stretch or shrink with movement of the operating channel, so as to prevent gas leakage. The operating channel is provided at the lower end with a duckbill anti-gas leakage flap, so as to prevent gas leakage. An angle is formed between the tail and the middle part of the sheath, which not only facilitates mounting the penetrating knife, but also prevents the penetrating knife from falling out during the surgery.

The beneficial effects of the present invention is as follows: the working casing pipe for minimally invasive surgery provided by the present invention can be penetrated directly into the abdominal cavity after carbon dioxide is injected, causing little injury to tissues and being safe; the hard semicircular penetrating casing pipe and the soft silicone channel are combined forming the working casing pipe, which has not only the largest apparatus operating space supported by the hard puncture outfit, but also the operational convenience provided by the soft puncture outfit; the semicircular middle part of the sheath allows large apparatus operating space; the instruments channels are scattered, for example, arranged up and down as viewed from the cross section, and can slide along the track, for example, slide left and right, so as to prevent collision between both hands; the two instrument channels are in staggered arrangement up and down (referring to the vertical direction in the cross section in the drawing), and the corresponding two sliding tracks are arranged in parallel, such that there is no interference among the instruments when a doctor operates with both hands, making the operation simple and convenient; the gasbag at the lower end of the silicone casing pipe prevents the working casing pipe from falling out of the incision.

### BRIEF DESCRIPTION OF THE DRAWINGS

The present invention will be further described below with reference to drawings and specific embodiments.

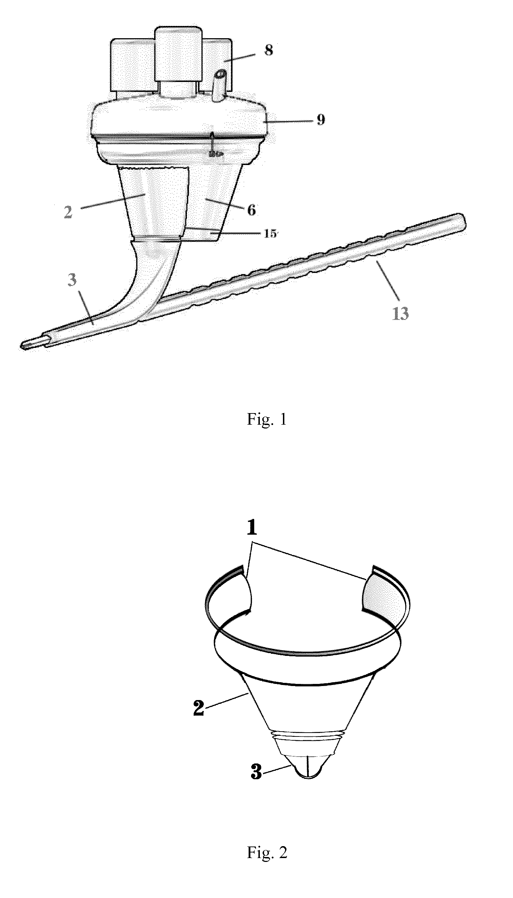

Fig. 1 is a schematic view of the working casing pipe with the penetrating channel according to an example of the present invention;
Fig. 1 is a schematic view of the structure of the circular sheath according to an example of the present invention;
Fig. 2 is a schematic view of the structure of the semicircular sheath according to an example of the present invention;
Fig. 3 is a schematic view of the structure of the multi-channel silicone packing and a schematic view of the connection between the cap and the sheath according to an example of the present invention;
Fig. 4 is a top view of the multi-channel cap and a schematic view of the structure of the multi-channel silicone packing according to an example of the present invention;
Fig. 5 is a schematic view of the structure of the instrument channels and track of the multi-channel cap according to an example of the present invention;
Fig. 7 is a schematic view of surgical penetration of the penetrating casing pipe according to an example of the present invention; and
Fig. 8 is a schematic view of pulling out the penetrating blade and rotating to put the penetrating casing pipe in place after the successful penetration according to an example of the present invention.

List of reference numbers: 1. An upper end of the sheath; 2. a middle part of the sheath; 3. a tail of the sheath; 4. an upper end of the sheath; 5. an insertion port of a penetrating knife; 6. a silicone casing pipe; 7. a carbon dioxide channel; 8. an instrument channel; 9. a multi-channel cap; 10. a channel track; 11. a multi-channel cushion; 12. an anti-gas leakage flap; 13. a penetrating knife; 14. an anti-reflux gas injection hole or valve of the gasbag on the cap; and 15. a gasbag.

### DETAILED DESCRIPTION OF THE EMBODIMENTS

As shown in Fig. 1 is a working casing pipe according to an example of the present invention, which comprises a sheath 2, a silicone casing pipe 6, a multi-channel cap 9, and a multi-channel cushion. Wherein the sheath 2 and the silicone casing pipe are fitted together, and the multi-channel cap and the silicone casing pipe are fitted together; the multi-channel cushion located under the multi-channel cap is not shown in Fig. 1, and will be discussed in the following.

Fig. 2 is a front view of the sheath 2, which includes the upper end 1, the middle part 2 and the tail 3. The upper end 1 of the sheath has a circular cross section.

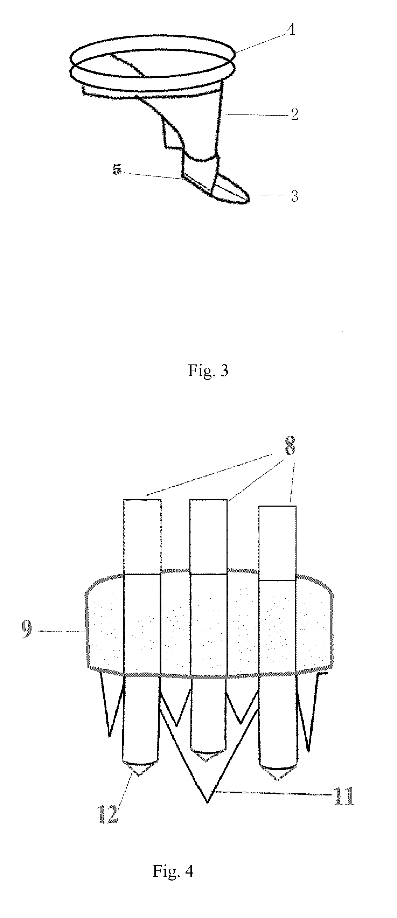

Fig. 3 is a side view of the sheath 2, the upper end 4 (1) of the sheath is circular, the cross section includes three parts, the middle part 2 of the sheath has a semicircular cross section, and the tail 3 of the sheath is shown to have a penetrating channel (5), i.e. a insertion port for a penetrating knife passing through.

Fig. 4 is a front schematic view of the connection between the multi-channel cushion 11 located at the upper part of the working channel, and the multi-channel cap 9 and the instrument channel 8. As shown in Fig. 4, the instrument channel 8 passes through the multi-channel cap 9 and the multi-channel cushion 11, the multi-channel cap is hard and can be, for example, made of hard plastics; the multi-channel cap is provided with multiple openings for letting the instrument channel 8 go through; the multi-channel cap 9 is provided below with a multi-channel cushion 11,which is soft, anti-gas leakage, and corrugation-shaped, with a V-shaped corrugation cross section shown in the drawing; the multi-channel cushion 11 can be made of anti-gas leakage silicone. In addition, the instrument channel 8 is provided at the tail with an anti-gas leakage flap 12, which can be, for example, a duckbill valve.

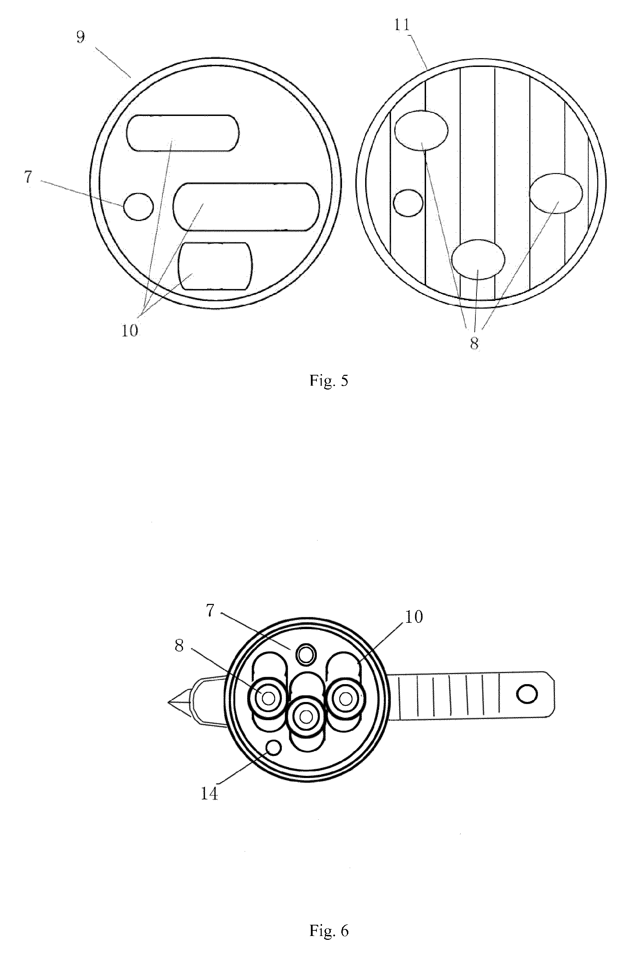

Fig. 5 is a top view of the multi-channel cap 9 and the multi-channel cushion 11 according to the present invention. As shown in the left drawing is a top view of the multi-channel cap 9, which includes an opening for letting the carbon dioxide channel 7 pass through and three instrument channel tracks 10. The three tracks 10 are parallel to each other, and the instrument channel 8 can pass through the track 10 and can also slide in the track 10 left and right. The right drawing is a top view of the multi-channel cushion, which is provided inside with a carbon dioxide channel and three instrument channels 8. It can be seen from the drawing that, in this example, each instrument channel track 10 is provided inside with an instrument channel 8, each of which can slide left and right along the respective track 10 as required by the surgical instrument operation. As required, three parallel tracks 10 are provided; the tracks 10 separated from and parallel to each other enable the instrument channels 8 to both move along the track, and keep a reasonable distance from each other while in moving; as shown in the right drawing, the three instrument channels 8 are scattered from each other, which prevents the instruments from interfering with and obstructing each other while in operation. The number and shape of the tracks 10 and the channels can be determined according to the actual needs of the surgery. For example, only two operating channels and two operating tracks can be provided for coordinating a bimanual operation. Again, for example, the operating tracks can be either in the form of parallel strips, or in the form of parallel or scattered semicircles, etc..

Fig. 6 is a top view of the multi-channel cap according to the present invention. It can be seen from Fig. 5 that, multi-channel cap 9 is provided at the top with three instrument channel tracks 10, each of which is circular at both ends and rectangular in the middle; there is one instrument channel 8 passing through each of the tracks, and the instrument channel 8 can move up and down in the track 10 (in the up-and-down direction in the drawing). In addition, the multi-channel cap 9 is further provided with a carbon dioxide channel 7 and a gas injection hole 14. The gas injection hole 14 can also be set as an anti-reflux gas injection valve for filling gas into the gasbag 15 at the lower end of the silicone casing pipe 6 as shown in Fig. 1, so as to prevent the working casing pipe from falling out of the incision. The drawing also schematically shows the penetrating knife and sheath.

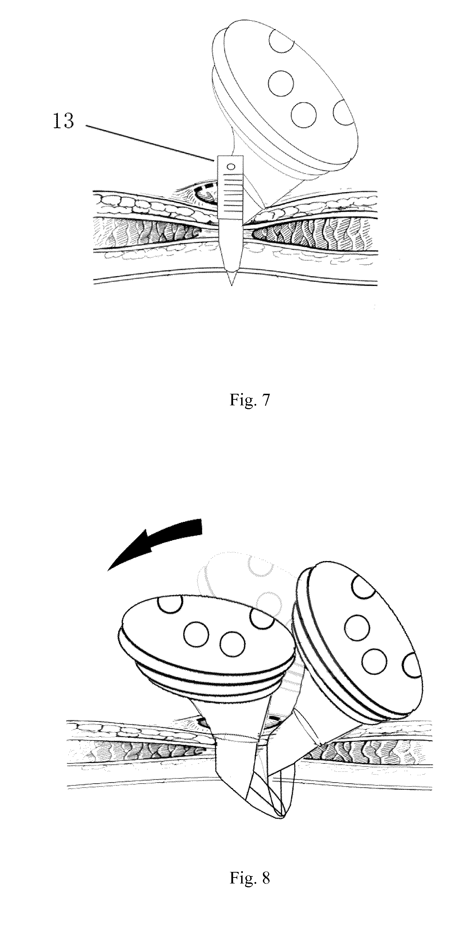

Fig. 7 and Fig. 8 are a schematic view of an example according to the present invention. As shown in Fig. 7, when the working casing pipe of the present invention is used in a single port laparoscopic surgery, the multi-channel semicircular penetrating casing pipe with the penetrating channel is used for the surgical penetration. While in use, after carbon dioxide gas is injected into the abdominal cavity by using the Veress needle, a penetrating knife 13 is inserted into the tail of the sheath of the multi-channel semicircular working casing pipe with the penetrating channel (Fig. 1), and penetrated into the abdominal wall. Fig. 8 shows that, after the working casing pipe in Fig. 7 is penetrated into the abdominal cavity, the penetrating knife 13 is pulled out, the casing pipe of the present invention is put in place and rotated into the abdominal cavity, and then the surgery begins.

In this way, not only the safety of surgery can be ensured, but also the injury to the abdominal wall and organs can be reduced, with the appearance of the wound also taken into account. It overcomes the current defect that the single port penetrating casing pipe can be put inside only after each layer of tissues is incised by surgery. Besides, the sheath 2 is hard and has support function, and the silicone casing pipe 6 is flexible. Therefore, the instrument has both larger operating space and operational flexibility. The instrument channels 8, viewed from the cross section, are scattered, such as the up and down staggered distribution in view of cross section, as shown in Fig. 4. In addition, there is a lateral oval track on the multi-channel cap, and the instrument channels can slide in the track. Therefore, when a doctor operates bimanualy the instrument(s) in left hand does not interfere with that/those in right hand, thus preventing the instruments from intersecting and colliding with each other, making the operation easy and convenient, improving flexibility of operation by both hands. A convenient minimally invasive surgery device is thus provided for a doctor to complete highly difficult surgery.

## Claims

1. A working casing pipe for minimally invasive surgery, **characterized in that**: it comprises a sheath, a silicone casing pipe, a multi-channel cap, and a multi-channel cushion.

2. The working casing pipe according to claim 1, **characterized in that**: the tail of the sheath at the lower end is provided inside with a penetrating channel to form an insertion port for a penetrating knife.

3. The working casing pipe according to claim 1 or 2, **characterized in that**: the middle part of the sheath is hard, and the silicone casing pipe is soft; and the middle part of the sheath and the silicone casing pipe are fitted together to form the middle part with both hard and soft materials, of the working casing pipe.

4. The working casing pipe according to claim 1 or 2, **characterized in that**: the middle part of the sheath has a semicircular cross section, an angle α is formed between the middle part and the tail of the sheath.

5. The working casing pipe according to claim 1 or 2, **characterized in that**: the multi-channel cap is provided with a carbon dioxide channel, multiple instrument channels and multiple channel tracks.

6. The working casing pipe according to claim 5, **characterized in that**: the multiple channel tracks are parallel to each other, and the apparatus channel can slide along the apparatus channel tracks.

7. The working casing pipe according to claim 1 or 2, **characterized in that**: the silicone casing pipe is provided at the lower end with a gasbag.

8. The working casing pipe according to claim 5, **characterized in that**: the multi-channel cap includes an anti-reflux gas injection hole or valve, to fill gas into the gasbag at the lower end of the silicone casing pipe.

9. The working casing pipe according to claim 1, **characterized in that**: the multi-channel cushion is a soft elastomer, and is provided at the upper end with the carbon dioxide channel and multiple instrument channels.

10. The working casing pipe according to claim 1 or 8, **characterized in that**: the multi-channel cushion is corrugation-shaped, and has a V or U-shaped cross section.

11. The working casing pipe according to claim 5 or 9, **characterized in that**: the multiple instrument channels are scattered;

12. The working casing pipe according to claim 9, **characterized in that**: the instrument channel is provided at the lower end with a duckbill anti-reflux flap.
